Europäisches Patentamt

European Patent Office

Office européen des brevets

(11) Publication number: **0 407 959 A2**

# EUROPEAN PATENT APPLICATION

(21) Application number: **90113135.9**

(51) Int. Cl.5: **C12P 7/64**

(22) Date of filing: **10.07.90**

(30) Priority: **11.07.89 JP 179674/89**
**09.11.89 JP 291719/89**

(43) Date of publication of application:
**16.01.91 Bulletin 91/03**

(84) Designated Contracting States:
**AT BE CH DE DK ES FR GB GR IT LI LU NL SE**

(71) Applicant: **LION CORPORATION**
**3-7, Honjo 1-chome**
**Sumida-ku Tokyo(JP)**

(72) Inventor: **Kitano, Kyozo**
**No. 1-3-402 Akitsu 2-chome**
**Narashino-shi, Chiba-ken(JP)**
Inventor: **Iwasaki, Ryozo**
**No. 5-18-404 Takahama 3-chome**
**Chiba-shi, Chiba-ken(JP)**
Inventor: **Mori, Nobuhiro**
**Esteito Suzuki 103, No. 1-3-10 Higashiowada**
**Ichikawa-shi, Chiba-ken(JP)**
Inventor: **Sasamoto, Hisashi**
**Ookubo-ryo, No. 14-2 Ookubo 3-chome**
**Narashino-shi, Chiba-ken(JP)**
Inventor: **Akamatsu, Taku**
**No. 144-42 Minamihonjuku-cho, Asahi-ku**
**Yokohama-shi, Kanagawa-ken(JP)**

(74) Representative: **Vossius & Partnerner**
**Siebertstrasse 4 P.O. Box 86 07 67**
**D-8000 München 86(DE)**

(54) Process for producing polyol fatty acid monoesters.

(57) A polyol fatty acid monoester can be produced with an industrial advantage at high synthesis ratio upon (1) mixing and reacting a saturated or unsaturated fatty acid having 6 to 22 carbon atoms or an ester of such a fatty acid with a lower alcohol having 1 to 3 carbon atoms and a polyol selected from glycerol and its derivatives, polyglycerol and its derivatives, ethylene glycol, polyethylene glycol, propylene glycol and polypropylene glycol or (2) mixing and reacting a glyceride oil or fat comprising a saturated or unsaturated fatty acid having 6 to 22 carbon atoms as the constituent fatty acid and one or more of the above polyols, wherein an enzymatic reaction is conducted by using an immobilized thermostable lipase under the presence of a secondary or tertiary alcohol.

## PROCESS FOR PRODUCING POLYOL FATTY ACID MONOESTERS

The present invention relates to a process for producing polyol fatty acid monoesters capable of synthesizing the monoesters by an enzymatic reaction using lipase in a short period of time at high synthesis ratio and with high content.

It has been known that lipase is an enzyme of hydrolyzing a fat or an ester of higher fatty acid and also that lipase causes a reaction reverse to the hydrolysis under appropriate conditions thereby synthesizing a polyol fatty acid ester.

As an enzymatic production process for a fatty acid monoglyceride by utilizing the reaction reverse to hydrolysis with lipase, there has been proposed a method of synthesizing a glyceride from a fatty acid ester of lower alcohol and glycerol by using lipase derived from Candida cylindracea (refer to Japanese Patent Laid-Open Sho 59-118094 and 59-118095), a method of synthesizing glyceride from a fatty acid or fatty acid ester of lower alcohol and glycerol by using lipase derived from Penicillium cyclopium (refer to Japanese Patent Laid-Open Sho 61-181390) or a method of synthesizing a glyceride from oil and fat and glycerol by using an alkaline lipase (Japanese Patent Laid-Open Sho 60-102192).

Further, as another enzymatic production process for a polyol fatty acid ester by utilizing the reaction reverse to hydrolysis with lipase, there has been known a method of synthesizing a propylene glycol fatty acid ester from a fatty acid or fatty acid ester of lower alcohol and propylene glycol by using lipase derived from Candida cylindracea (refer to Japanese Patent Laid-Open Sho 61-149099) or a method of synthesizing a polyglycerol fatty acid ester from a fatty acid and a polyglycerol with an average polymerization degree of 3 or greater (Japanese Patent Laid-Open Sho 61-187795).

However, in any of the methods described above, the reaction is conducted in an aqueous emulsion system and, since the reaction products obey the rule of indiscriminate distribution (Emulsifier for Foods, written by Tohru Hidaka, Pages 13 to 16), reaction products even in the case of synthesizing a monoglyceride of a fatty acid also comprises a mixture of glycerol, monoglyceride, diglyceride, triglyceride and polyglyceride and it is difficult to obtain only the monoglyceride at high purity. Accordingly, the polyol fatty acid monoester can be formed only at a low content and at a poor yield in the reaction product.

Further, although the reaction using the lipase proceeds at a low temperature and a polyol fatty acid monoester of excellent quality with no thermal degradation can be obtained by the method described above, there is a problem that since the reaction rate is extremely low as compared with usual chemical reaction, it takes a long production time and large reactor is necessary to increase the cost even if inexpensive lipase is utilized, thus making industrial application difficult. In addition, there is also a drawback that the superiority to the chemical reaction can not be ensured unless the monoester content is high.

As a reaction in the non-emulsion system, there has also been proposed a method of using lipase derived from Chromobacterium viscosum and conducting ester synthesis at the interface between fatty acid and glycerol (JAOCS., Vol. 61, No. 4, April, 1984, Pages 776 to 781) or a method of synthesizing an ester by reacting a bacterial alkaline lipase under the presence of an organic solvent (Japanese Patent Laid Open Sho 61-257191). Although such a method can attain high synthesis ratio, it requires a reaction for long time such as more than one day, to obtain synthesis ratio of higher than 90% based on a reaction rate of fatty acid or fatty acid ester and lower alcohol with 1 to 3 carbon atoms.

As described above, since conventional synthesis processes using lipase involve the problems in view of reaction time, synthesis ratio, etc., it is difficult to synthesize polyol fatty acid monoesters with an industrial advantage.

It is an object of the present invention to provide a process for producing a polyol fatty acid monoester by using an enzymatic reaction of lipase capable of synthesizing the monoester in a short period of time at a high synthesis ratio and with high monoester content in the reaction products.

The present inventor has made an earnest study for attaining the foregoing object and, as a result, has found that a polyol fatty acid monoester can be produced with an industrial advantage at high synthesis ratio upon (1) mixing and reacting a saturated or unsaturated fatty acid having 6 to 22 carbon atoms or an ester of such a fatty acid with a lower alcohol having 1 to 3 carbon atoms and a polyol selected from glycerol and its derivatives, polyglycerol and its derivatives, ethylene glycol, polyethylene glycol, propylene glycol, and polypropylene glycol or (2) mixing and reacting a glyceride oil or fat comprising a saturated or unsaturated fatty acid having 6 to 22 carbon atoms as the constituent fatty acid and one or more of the above polyols, wherein an enzymatic reaction is conducted by using an immobilized thermostable lipase under the presence of a secondary or tertiary alcohol, particularly, at a temperature higher than 40°C, while removing by-products, preferably, such that the concentration of water or lower alcohol having 1 to 3 carbon atoms by produced in the enzymatic reaction is not greater than 0.5% by weight in the reaction system, whereby the reaction proceeds in a short period of time and a polyol fatty acid monoester can be obtained with high content of the monoester in the reaction product.

Accordingly, in the first aspect of the present invention, there is provided a process for producing a polyol fatty and monoester, wherein a thermostable immobilized lipase is acted under the presence of a secondary alcohol and/or tertiary alcohol on a mixture of a saturated or unsaturated fatty acid having 6 to 22 carbon atoms or an ester of such a fatty acid with a lower alcohol having 1 to 3 carbon atoms and a polyol selected from glycerol and its derivatives, polyglycerol and its derivatives, ethylene glycol, polyethylene glycol, propylene glycol, and polypropylene glycol.

In the second aspect of the present invention, there is provided a process for producing a polyol fatty acid monoester, wherein a heat-resistant immobilized lipase is acted under the presence of a secondary alcohol and/or tertiary alcohol on a mixture of a glyceride oil or fat comprising a saturated or unsaturated fatty acid having 6 to 22 carbon atoms as the constituent fatty acid and a polyol selected from glycerol and its derivatives, polyglycerol and its derivatives, ethylene glycol, polyethylene glycol, propylene glycol, and polypropylene glycol, thereby conducting an ester exchanging reaction between them.

Fig. 1 is a schematic view of a device used for producing a glycerol fatty acid monoester by using an enzyme column, and

Fig. 2 is a graph illustrating the change of the amount of monoester formed with elapse of time in a case of continuously producing a glycerol fatty acid monoester for a long period of time in the device shown in Fig. 1.

In a process for producing a polyol fatty acid monoester according to the first aspect of the present invention, a fatty acid or its ester and a polyol are used as the starting material. Further, in the second aspect of the present invention, a glyceride oil or fat and polyol are used as the starting material.

The fatty acid used in the present invention is a saturated or unsaturated linear or branched fatty acid with 6 to 22 carbon atoms which may be substituted with hydroxy group, carbonyl group, phenyl group, etc. Specifically, the fatty acid usable herein can include, for example, caproic acid, sorbic acid, enanthic acid, caprylic acid, pelargonic acid, caprinic acid, undecylic acid, lauric acid, tridecanoic acid, myristic acid, pentadecanoic acid, palmitoleic acid, palmitinic acid, margaric acid, stearic acid, isostearic acid, oleic acid, linolic acid, linolenic acid, nonadecanoic acid, eicosanic acid, docosanic acid, docosenic acid, arachidonic acid, ricinoleic acid and dihydroxystearic acid.

Further, as the fatty acid ester, esters of the fatty acid with 6 to 22 carbon atoms as described above and lower alcohols with 1 to 3 carbon atoms, for example, methanol, ethanol and propanol are used. Specifically, there can be mentioned, for example, methyl capronate, ethyl capronate, methyl caprinate, ethyl caprinate, methyl laurate, ethyl laurate, propyl laurate, methyl myristate, ethyl myristate, propyl myristate, methyl palmitate, ethyl palmitate, propyl palmitate, methyl stearate, ethyl stearate, propyl stearate, methyl oleate, ethyl oleate, propyl oleate, methyl linoleate, ethyl linoleate, propyl linoleate, methyl linoleate, ethyl linolenate, propyl linolenate, methyl eicosanate, methyl arachidonate, methyl docosanate and methyl docosenate.

The fatty acid constituting the glyceride oil or fat used in the present invention is a saturated or unsaturated linear or branched fatty acid with 6 to 12 carbon atoms which may be substituted with hydroxy group, carbonyl group, phenyl group, etc., for which the fatty acid as described above can be exemplified.

Specifically, as the glycerol oil and fat, there can be mentioned, for example, edible burdock seed oil, cherry seed oil, pomegranate seed oil, safflower oil, watermelon seed oil, soybean oil, soybean germ (embryo) oil, tabacco seed oil, surflower seed oil, sun tree (Japanese cypress) seed oil, mowah oil, barley oil, barley bran oil, barley germ oil, pumpkin seed oil, sorghum oil, sesame oil, wheat oil, millet oil, corn oil, rape seed oil, carrot seed oil, rice bran oil, loofah seed oil, spinach seed oil, mandarin oil, cotton seed oil, rhy germ oil, apple seed oil, lemon seed oil, olive oil, olive hernel oil, coffee bean oil, peanuts oil, laurier nuts oil, camellia oil, castor oil, nutmeg fat, palm oil, palm hernel oil, coconut oil, beef tallow, lard, horse tallow, pig external oil, pig perinephric fat, mutton tallow, buck tallow, quil tallow, goose tallow, pheasant oil, chicken liver oil, chicken leg oil, wild duck (mallard) oil, locust oil, chrysalis oil, tortoise oil, bullfrog oil, terrapin oil, sardine oil, eel oil, bonito oil, salmon oil, mackerel oil, saury oil, herring oil, rainbow trout oil, yellowtail oil, atka mackered oil, tuna oil, trout oil, shark liver oil, ray oil, silver shark liver oil, etc., with no particular restriction thereto.

In the present invention, one or more of polyols selected from glycerol and its derivatives, polyglycerol and its derivatives, ethylene glycol, polyethylene glycol, propylene glycol and polypropylene glycol can be used. The derivatives of glycerol include organic acid glycerol such as acetic acid glycerol, lactic acid glycerol, citric acid glycerol and succinic acid glycerol, sulfonated glycerol, ethoxylated glycerol, and phosphated glycerol. The derivatives of polyglycerol include organic acid glycerol, sulfonated glycerol, ethoxylated glycerol, and phosphated glycerol. The polyglycerol should preferably have an average polymerization degree of 2 to 22, more preferably 2 to 10. Polyethylene glycol and polypropylene glycol should preferably have an average polymerization degree of 2 to 40, more preferably 2 to 20.

The mixing ratio of the fatty acid or its ester and the polyol as described above is preferably form 0.1 to 10 mol and, more preferably, 1 to 3 mol of the polyol based on one mol of the fatty acid or its ester.

The mixing ratio of the glyceride oil or fat and the polyol described above is preferably from 0.2 to 20 mol, more preferably, 2 to 6 mol of the polyol based on one mol of the glyceride oil or fat.

In the present invention, an immobilized thermostable lipase is used for reacting the starting materials described above by utilizing the enzymatic reaction.

Various kinds of lipases can be used as the thermostable lipase so long as they have such heat resistance as possessing greater than 40%, preferably, greater than 80% and, more preferably, greater than 95% of the residual activity after dissolving 50 mg of a lipase powder into 0.4 ml of a phosphoric acid buffer (0. 1M, pH7) and then heating at 70°C for 30 min. For instance, thermostable lipase derived from Candida antarctica (sp-382, manufactured by NOVO Co.), thermostable lipase derived from Mucor miehei (Lipozyme, manufactured by NOVO Co.), etc. are suitable since they have excellent heat resistance as apparent from the result of experiments described later, with no restriction only thereto.

As a method of immobilizing the thermostable lipase as described above, any of the methods of carrier bonding, crosslinking and inclusion may be used and, in particular, the carrier bonding method can be adopted suitably.

In this case, as an immobilizing carrier, there can be mentioned, specifically, those inorganic materials such as active carbon, porous glass, acidic white clay, bleached white clay, kaolinite, alumina, silica gel, bentonite, hydroxyapatite, potassium phosphate and other metal oxides, natural polymeric compounds such as starch and gluten, synthetic polymeric materials such as polyethylene, polypropylen, phenol-formalin resin, acrylic resin, anionic exchange resin and cationic exchange resin. In particular, synthetic polymeric material having porosity as the physical form, for example, porous polyethylene, porous polypropylene, porous phenol formalin resin, porous acrylic resin are used most preferably. In the present invention, various immobilizing carriers other than above may also be used so long as they do not hinder the development of the enzymatic reactivity.

Further, as the amount of lipase immobilized on the immoibilizing carrier, it is preferred that a protein from 0. 1 to 500 mg is immobilized to one gram of the immobilizing carrier and, in particular, a protein containing about 1 to 50 % by weight of lipase in the protein is suitably immobilized.

The amount of the thermostable immobilized lipase used has no particular restrictions, although the amount can preferably be from 0. 1 to 10,000 parts by weight, more preferably, from 1 to 2,000 parts by weight based on 100 parts by weight of the fatty acid or its ester.

In the present invention, an enzymatic reaction of the fatty acid or its ester or a glyceride oil or fat with the polyol by using a thermostable immobilized lipase is conducted under the presence of a secondary or tertiary alcohol. The secondary or the tertiary alcohol can easily dissolve the fatty acid or its ester, the glyceride oil or fat and the polyol, and a polyol fatty acid monoester can be synthesized efficiently by conducting the reaction under the presence of the secondary or the tertiary alcohol.

As the secondary or the tertiary alcohol, there can be mentioned, for example, 2, 4-dimethyl-3-pentanol, 2,6-dimethyl-4-heptanol, tertiary butyl alcohol, tertiary amyl alcohol, diacetone alcohol, 3- methyl-3-pentanol, 3-ethyl-3-pentanol, 3-propyl-3-pentanol, 2-methyl-2-hexanol. and 2-ethyl-2-hexanol. The alcohols may be used singly or in combination of two or more of them.

The amount of the secondary or the tertiary alcohol used depends on the kind of the secondary or tertiary alcohol used, carbon chain length of the ester, the kind of the glyceride oil and fat, the kind of the polyol, reaction temperature, etc. and preferably, it is from 10 to 99 % by weight, in particular, 60 to 80 % by weight of the entire reaction system. If the amount of the secondary or the tertiary alcohol used is less than 10 % by weight, it can not sufficiently dissolve the fatty acid or its ester, glyceride oil or fat and polyol and it may be difficult to synthesize a polyol fatty acid monoester efficiently. If it exceeds 99 % by weight, the concentration of the substrate (fatty acid or its ester, glyceride oil or fat and polyol) relative to the solvent is lowered to result in the reduction of the enzymatic reaction rate.

Further, in the present invention, other organic solvents than the secondary or the tertiary alcohol described above may be mixed together within such a range as not hindering the reaction of the present invention. In this case, there can be mentioned, as other organic solvents, for example, aromatic hydrocarbons such as benzene, toluene, xylene and phenol, ketones such as acetone, aliphatic hydrocarbons such as n-hexane and iso-octane, cycloaliphatic hydrocarbons such as cyclopentane and cyclohexane, ethers such as dimethyl ether, diethyl ether and dioxane, and halogenated hydrocarbons such as carbon tetrachloride and chloroform. Although nitrogen-containing solvents such as pyridine, dimethyl formamide and quinoline and sulfoxide solvents such as dimethyl sulfoxide can homogenize the reaction system, they

4

may sometime reduce the enzyme stability of lipase and, accordingly, it is preferred not to mix them as the solvent in the present invention.

In the process for producing a polyol fatty acid monoester according to the present invention, a thermostable immobilized lipase is acted under the presence of a secondary alcohol and/or tertiary alcohol on a mixture of a saturated or unsaturated fatty acid having 6 to 22 carbon atoms, an ester of such a fatty acid and a lower alcohol having 1 to 3 carbon atoms or a glycerol oil or fat comprising the fatty acid described above as the constituent fatty acid, and the polyol as defined above.

Upon enzymatic reaction by using a thermostable immobilized lipase, reaction conditions can be adjusted properly. It is preferred that the reaction is conducted at a temperature higher than 40°C, more preferably 40 to 60°C and the reaction can be completed within a short period of time of about 0.5 to 10 hours by conducting the reaction under such a temperature condition.

Further, upon producing a polyol fatty acid monoester by the process according to the present invention, it can be conducted by adopting, for example, a method of passing liquid substrate into a column packed with an immobilized lipase (packed column system), a method of introducing a liquid substrate and an immobilized enzyme into a reaction vessel and then conducting reaction by stirring and shaking (batchwise system), a method of continuously conducting the reaction by a batchwise system (continuous stirring vessel system), etc.

In the process according to the present invention using a fatty acid or its ester, water or lower alcohol with 1 to 3 carbon atoms is by-produced by the enzymatic reaction, in which it is preferred to remove the by-products such that the concentration of the by-products in the system is less than 0.5 % by weight and, particularly, less than 1 % by weight, for efficiently proceeding the reaction. As a method of removing the by-products, there can be mentioned, for example, a method of removing by adsorption using zeolite, molecular sieve or sodium sulfate, a method of introducing dry air or inert gas into a reaction vessel and removing the by-products by evaporization into gases, or a method of reducing the pressure in the reaction vessel and then evaporizing the by-products out of the reaction vessel. By properly combining the removing method with the enzymatic reaction device as described above, synthesis reaction can be conducted at high efficiency.

On the other hand, water and lower alcohol having 1 to 3 carbon atoms are not by-produced in the reaction process using a glyceride oil or fat. However, if the substrates and solvents contain much water, it is desirable to keep the water content in the reaction system to less than 0.5 % by weight, preferably, 0. 1 % by weight, in order to prevent hydrolysis of glyceride oil and fat by lipase.

After the completion of the reaction, a polyol fatty acid monoester in the reaction products can be separated and collected by a customary method.

The present invention will be described more specifically referring to examples but the invention is not restricted only to the following examples.

Example 1

Thermostable test for various lipases

Various kinds of powdery lipases were dissolved each by 50 mg into 0.4 ml of a phosphoric acid buffer (0. 1 M, pH 7) and then heated at 70°C for 30 min. After cooling it with chilled water, it was dissolved to 5 g of glycerol to which 0.5 g of oleic acid was added and an enzymatic reaction was conducted at 30°C for one hour. After the reaction was over, 20 ml of an acetone/ethanol solution (volume ratio = 1 : 1) gas added to terminate the enzymatic reaction. As a blank, a not-heated aqueous lipase solution was used and an enzymatic reaction was conducted in parallel.

After terminating the enzymatic reaction, it was titrated with a 0. 1 M ethanol/potassium hydroxide solution and the enzymatic activity was calculated based on the reduction of the fatty acid and the relative activity before and after the heating was determined according to the following equation:

$$\text{Relative activity (\%)} = \frac{\text{Enzymatic activity after heating}}{\text{Enzymatic activity before heating}} \times 100$$

Ester synthesizing performance of various immobilized lipases in solution

To a mixture of 1 g (3.5 mM) of oleic acid and 0.98 g (10.5 mM) of glycerol, were added 5 ml of tertiary butyl alcohol and, further, 100 mg of each of lipases shown in Table 1 immobilized into a porous acrylic resin. Then, 1 g of molecular sieve 3A (manufactured by Wako Junyaku Co.) was added as a dehydrating agent and reacted at 50°C for 6 hours under stirring. After the reaction was over, it was filtered under a reduced pressure to remove the immobilized lipase and the molecular sieve 3A. The reaction filtrate was titrated with 0. 1 M ethanol/potassium hydroxide solution and the ester synthesizing rate was calculated based on the reduction of oleic acid in accordance with the following equation:

$$\text{Ester synthesizing rate (\%)} = \frac{\text{Oleic acid before enzymatic reaction (g)} - \text{Oleic acid after enzymatic reaction (g)}}{\text{Oleic acid before enzymatic reaction (g)}} \times 100$$

The results are shown together in Table 1.

Table 1

| Lipase | | Retative activity (%) | Ester synthesis rate (%) |
|---|---|---|---|
| Origin | Commercial name (manufacturer's name) | | |
| Candida antarctica | sp-382 (NOVO Co.) | 98 | 96 * |
| Mucor miehei | Lipozyme (NOVO Co.) | 95 | 83 ** |
| Mucor sp | sp-225 (NOVO Co.) | 0 | 0 |
| Mucor sp | sp-356 (NOVO Co.) | 0 | 0 |
| Mucor sp | sp-400 (NOVO Co.) | 0 | 10 |
| Rhizodpus delemer | Lipase (Seikagaku Kogyo Co., Ltd.) | 6 | 26 |
| - | Lipase (Nagase Industrial Co., Ltd.) | 0 | 0 |
| - | Lipase (Toyo Jozo Co., Ltd.) | 0 | 17 |
| - | Talipase (TanabeSeiyaku Co., Ltd.) | 0 | 0 |
| Candida rugosa | Lipase MY (Meito Sangyo Co., Ltd.) | 0 | 0 |
| Candida rugosa | Lipase DF (Meito Sangyo Co., Ltd.) | 3 | 0 |
| Alcaligenes sp | Lipase PL (Meito Sangyo Co., Ltd.) | 11 | 31 |
| Pseudomonas fluorescens | Lipase P (Amano Seiyaku Co., Ltd.) | 8 | 21 |
| Penicillium aurantiogriseum | Lipase G (Amano Seiyaku Co., Ltd.) | 0 | 0 |
| Aspergillus niger | Lipase AP (Amano Seiyaku Co., Ltd.) | 0 | 0 |
| Rhizopus sp | Saiken (Osaka Saikin Laboratory) | 0 | 0 |
| Rhizopus japonicus | Saiken (Osaka Saikin Laboratory) | 0 | 0 |
| Arthrobacter sp | BSL (Godo Shusei Co., Ltd.) | 0 | 0 |
| Rhizopus arrhizus | Lipase (Sigma Co.) | 0 | 0 |
| Porcine pancreas | Lipase (Sigma Co.) | 0 | 0 |

*: Refer to Example 2,
**: Refer to Example 18

From the result shown in Table 1, it can be seen that Lipase sp-382 derived from Candida antarctica and Lipozyme derived from Mucor miehei, being immobilized, have excellent heat resistance and can attain a high ester synthesis rate at 50°C which is relatively high reaction temperature for the lipase reaction and in a shorter period of time.

Example 2

After adding 5 ml of tertiary butyl alcohol and, further, 100 mg of thermostable lipasederived from Candida antarctica immobilized on an acrylic resin (hereinafter referred to as immobilized lipase sp-382) to a mixture of 1 g (3.54 mM) of oleic acid and 0.98 g (10.6 mM) of glycerol, 1 g of molecular sieve 3A was added as a dehydrating agent and reacted at 50°C for 6 hours under stirring. After the reaction was over, it was filtered to remove the immobilized lipase sp-382 and the molecular sieve 3A.

Then, 10 $\mu$l of the reaction filtrate was taken into 1 ml screw tube, to which were added 40 ml of pyridine and, further, 50 $\mu$l of pyridine incorporated with n-tetradecane as an internal standard material (20 mg/ml) and 200 μl of N,O-bis(trimethylsilyl) trifluoroacetoamido as a silylating agent and they were reacted while being kept stationary at 70 to 80°C for 30 min. den 1 μl of the reaction solution was analyzed on gas chromatography to measure-the percent by weight of unreacted fatty acid and synthesized glycerol monoester, diester and triester in the oil, the ester synthesis ratio was 96.0%, the reaction product contained 86.9% of glycerol monooleate and 9.0% of glycerol dioleate, no glycerol trioleate being detected.

Example 3

1.05 g (3.54 mM) of methyl oleate, 0.98 g (10.6 mM) of glycerol, 5 ml of tertiary butyl alcohol, 100 mg of immobilized lipase sp-382 and 1 g of molecular sieve 5A were taken into a 30 ml volume Erlenmeyer flask, which were shaken at 70°C for 6 hours to react and then processed in the same procedures as those in Example 2 to analyze glycerol ester. As a result, the ester synthesis ratio was 98.2% and the reaction product contained 88.9% of glycerol monooleate and 9.3% of glycerol dioleate.

Example 4

1. 10 g (3.54 mM) of ethyl oleate, 0.98 g (10.6 mM) of glycerol, 5 ml of tertiary butyl alcohol, 100 mg of immobilized lipase sp-382 and 1 g of molecular sieve 5A were taken into a 30 ml volume Erlenmeyer flask, which were shaken to react at 90°C for 3 hours and then processed and analyzed in the same procedures as those in Example 2. As a result, the ester synthesis ratio was 99. 1% and the reaction product contained 89.6% of glycerol monooleate and 9.5% of glycerol dioleate.

Example 5

1.05 g (3.54 mM) of methyl oleate, 5 ml of tertiary butyl alcohol, 100 mg of immobilized lipase sp-382 and 1 g of molecular sieve 5A were charged into 30 ml volume Erlenmeyer flask, to which glycerol was added each in an amount of 0.38 g (3.54 mM), 0.98 g (10.6 mM) and 1.63 g (17.7 mM) respectively, and they were reacted at 50- for 6 hours. After the reaction was over, the ingredient contents for the unreacted methyl oleate and the synthesized glycerol oleate were determined. The results are shown in Table 2.

Table 2

| Glycerol/methyl oleate (molar ratio) | Ester synthesis ratio (%) | Ingredient ratio (%) | | | |
|---|---|---|---|---|---|
| | | Glycerol monooleate | Glycerol dioleate | Glycerol trioleate | Methyl oleate |
| 1 | 93.5 | 74.8 | 18.7 | 0 | 5.5 |
| 3 | 96.3 | 88.0 | 8.3 | 0 | 1.7 |
| 5 | 97.0 | 88.3 | 8.7 | 0 | 0.9 |

From the results shown in Table 2, it can be seen that the ester synthesis ratio and the content of glycerol monooleate are improved along with the increase of the molar ratio of glycerol/methyl oleate.

Examples 6 and 7

0.99 g (3.54 mM) of linolic acid, 0.98 g (10.6 mM) of glycerol, 10 ml of tertiary butyl alcohol, 100 mg of immobilized lipase sp-382 and 1 g of molecular sieve 3A were charged into 30 ml volume Erlenmeyer flask and then reacted at 50°C for 6 hours under shaking. As a result, the ester synthesis ratio was 96.3% and the reaction product contained 86.4% of glycerol monolinorate and 9.8% of glycerol dilinorate.

When the same reaction as described above was conducted except for replacing linolic acid with

8

arachidonic acid, the ester synthesis ratio was 94.6% and the reaction product contained 91.5% of glycerol monoester and 3.2% of glycerine diester.

Example 8

0.96 g (3.54 mM) of methyl palmitate, 0.98 g (10.6 mM) of glycerol, 7 ml of tertiary butyl alcohol, 100 mg of immobilized lipase sp-382 and 1 g of molecular sieve 3A were charged into 30 ml volume Erlenmeyer flask and then reacted at 50°C for 6 hours under shaking. As a result, the ester synthesis ratio was 96.0% and the reaction product contained 88.3% of glycerol monopalmitate and 7.7% of glycerol dipalmitate.

Example 9

0.81 g (3.54 mM) of myristic acid, 0.98 g (10.6 mM) of glycerol, 3 ml of tertiary butyl alcohol, 100 mg of immobilized lipase sp-382 and 1 g of molecular sieve 3A were charged into 30 ml volume Erlenmeyer flask and then reacted at 60°C for 4 hours under shaking. As a result, the ester synthesis ratio was 96.4% and the reaction product contained 85.0% of glycerol monomyristate and 11.4% of glycerol dimyristate.

Example 10

0.76 g (3.54 mM) of methyl laurate, 0.98 g (10.6 mM) of glycerol, 5 ml of tertiary butyl alcohol, 100 mg of immobilized lipase sp-382 and 1 g of molecular sieve 3A were charged into 30 ml volume Erlenmeyer flask and then reacted at 60°C for 4 hours under shaking. As a result, the ester synthesis ratio was 96.2% and the reaction product contained 83.8% of glycerol monolaurate and 12.5% of glycerol dilaurate.

Example 11

0.61 g (3.54 mM) of capric acid, 0.98 g (10.6 mM) of glycerol, 5 ml of tertiary butyl alcohol, 100 mg of immobilized lipase sp-382 and 1 g of molecular sieve 3A were charged into 30 ml volume Erlenmeyer flask and then reacted at 70°C for 4 hours under shaking. As a result, the ester synthesis ratio was 97. 1% and the reaction product contained 85.2% of glycerol monocaprate and 11.9% of glycerol dicaprate.

Example 12

0.46 g (3.54 mM) of methyl capronate, 0.98 g (10. 6 mM) of glycerol, 5 ml of tertiary butyl alcohol, 100 mg of immobilized lipase sp-382 and 1 g of molecular sieve 3A were charged into 30 ml volume Erlenmeyer flask and then reacted at 70°C for 4 hours under shaking. As a result, the ester synthesis ratio was 97.2% and the reaction product contained 83.7% of glycerol monocapronate and 13.5% of glycerol dicapronate.

Example 13

0.76 g (3.54 mM) of pentadecanoic acid, 0.98 g (10.6 mM) of glycerol, 5 ml of tertiary butyl alcohol, 100 ml of immobilized lipase sp-382 and 1 g of molecular sieve 3A were charged into 30 ml volume Erlenmeyer flask and then reacted at 50°C for 6 hours under shaking. As a result, the ester synthesis ratio was 97.6% and the reaction product contained 88.2% of glycerol monopentadecanoate and 9.4% of glycerol dipentadecanoate.

Example 14

1.05 g (3. 54 mM) of methyl oleate, 0.98 g (10.6 mM) of glycerol, 5 ml of diacetone alcohol, 100 mg of

9

immobilized lipase sp-382 and 3 g of molecular sieve 5A were charged into 30 ml volume Erlenmeyer flask and then reacted at 40°C for 24 hours under shaking. As a result, the ester synthesis ratio was 94.7% and the reaction product contained 86.5% of glycerol monooleate and 8.3% of glycerol dioleate.

## Examples 15 and 16

1.05 g (3.54 mM) of methyl oleate, 0.98 g (10.6 mM) of glycerol, 10 ml of 2, 4-dimethyl-3-pentanol, 100 mg of immobilized lipase sp-382 and 1 g of molecular sieve 3A were charged into 30 ml volume Erlenmeyer flask and then reacted at 50°C for 6 hours under shaking. As a result, the ester synthesis ratio was 93.2% and the glycerol oleate contained 84.7% of glycerol mooleate and 8.4% of glycerol dioleate.

When the same reaction as described above was conducted except for replacing 2,4-dimethyl-3-pentanol with 3-methyl-3-pentanol, the ester synthesis ratio was 94.0% and the reaction product contained 85.1% of glycerol monooleate and 9.0% of glycerol dioleate.

## Example 17

0.96 g (3.54 mM) of methyl palmitate, 0.98 g (10.6 mM) of glycerol, 2.5 ml of tertiary butyl alcohol, 2.5 ml of diacetone alcohol, 100 mg of immobilized lipase sp-382 and 1 g of molecular sieve 5A were charged into 30 ml volume Erlenmeyer flask and then reacted at 60°C for 5 hours under shaking. As a result, the ester synthesis ratio was 94.7% and the reaction product contained 85.9% of glycerol monopalmitate and 8.7% of glycerol dipalmitate.

## Example 18

0.96 g (3.54 mM) of methyl palmitate, 0.98 g (10.6 mM) of glycerol, 4 ml of tertiary butyl alcohol, 1 ml of hexane, 100 mg of immobilized lipase sp-382 and 1 g of molecular sieve 5A were charged into 30 ml volume Erlenmeyer flask and then reacted at 60°C for 5 hours under shaking. As a result, the ester synthesis ratio was 92.9% and the reaction product contained 81.3% of glycerol monopalmitate and 11.6% of glycerol dipalmitate.

## Example 19

1 g (3.54 mM) of oleic acid, 0.98 g (10.6 mM) of glycerol, 5 ml of tertiary butyl alcohol, 100 mg of thermostable lipase derived from Mucor miehei and immobilized into a phenol-formalin resin (Lipozyme) and 1 g of molecular sieve 5A were charged into 30 ml volume Erlenmeyer flask and then reacted at 50°C for 6 hours under shaking. As a result, the ester synthesis ratio was 82.4% and the reaction product contained 75.7% of glycerol monooleate and 6.7% of glycerol dioleate.

## Example 20

0.76 g (3.54 mM) of methyl laurate, 1.37 g (7.08 mM) of sodium glycerol monosulfonate, 5 ml of diacetone alcohol, 100 mg of immobilized lipase sp-382 and 1 g of molecular sieve 5A were charged into 30 ml volume Erlenmeyer flask and then reacted at 50°C for 6 hours under shaking. As a result, the ester synthesis ratio 95.0% and the reaction product contained 93.8% of glycerol sulfonate monolaurate and 1.2% of glycerol sulfonate dilaurate.

## Example 21 to 23

1 g (3.54 mM) of oleic acid, 0.58 g (3.54 mM) of diglycerol, 5 ml of tertiary butyl alcohol, 100 mg of immobilized lipase sp-382 and 1 g of molecular sieve 3A were charged into 30 ml volume Erlenmeyer flask and then reacted at 50°C for 6 hours under shaking. As a result, the ester synthesis ratio was 96.8%, diglycerol monooleate was 88.4% and diglycerol dioleate was 8.4%.

When the same reaction as described above was conducted except for replacing diglycerol with 1.12 g (3.54 mM) of tetraglycerol or 2.69 g (3.54 mM) of decaglycerol, the ester synthesis ratios were 97.5% and 96.7%, respectively.

Example 24

0.91 g (3.54 mM) of palmitic acid, 2.69 g (3.54 mM) of decaglycerol, 5 ml of diacetone alcohol, 100 mg of immobilized lipase sp-382 and 1 g of molecular sieve 3A were charged into 30 ml volume Erlenmeyer flask and reacted at 50°C for 6 hours. As a result, the ester synthesis ratio was 96. 1%.

Examples 25 to 30

1.05 g (3.54 mM) of methyl oleate, 2.69 g (3.54 mM) of decaglycerol, 5 ml of tertiary butyl alcohol, 100 mg of immobilized lipase sp-382 and 1 g of molecular sieve 3A were charged into 30 ml volume Erlenmeyer flask and then reacted at 60°C for 6 hours under shaking. As a result, the ester synthesis ratio was 98.7%.

When the same reaction as described above was conducted except for replacing methyl oleate with 1.06 g (3.54 mM) of methyl stearate, 0.96 g (3.54 mM) of palmitic acid, 0.86 g (3.54 mM) of methyl myristate, 0.66 g (3.54 mM) of methyl capronate, 0.46 g (3.54 mM) of methyl capronate. The ester synthesis ratios were 97.4%, 96.8%, 98.2%, 98.5% and 99.1%, respectively.

Example 31

1g (3.54 mM) of oleic acid, 0.81 g (10.6 mM) of propylene glycol, 5 ml of tertiary butyl alcohol, 100 mg of immobilized lipase sp-382 and 1 g of molecular sieve 3A were charged into 30 ml volume Erlenmeyer flask and then reacted at 50°C for 6 hours under shaking. As a result, the ester synthesis ratio was 93.7% and the reaction product contained 92. 1% of propylene glycol monooleate and 1.6% of propylene glycol dioleate.

Example 32

0.99 g (3.54 mM) of linoleic acid, 0.81 g (10.6 mM) of propylene glycol, 5 ml of diacetone alcohol, 100 mg of immobilized lipase sp-382 and 1 g of molecular sieve 3A were charged into 30 ml volume Erlenmeyer flask and then reacted at 60°C for 6 hours under shaking. As a result, the ester synthesis ratio was 94.1% and the reaction product contained 93.0% of propylene glycol monolinoleate and 1.1% of propylene glycol dilinoleate.

Example 33

1.06 g (3.54 mM) of methyl stearate, 0.27 g (3.54 mM) of propylene glycol, 5 ml of tertiary butyl alcohol, 100 mg of immobilized lipase sp-382 and 1 g of molecular sieve 3A were charged into 30 ml volume Erlenmeyer flask and then reacted at 70°C for 4 hours under shaking. As a result, the ester synthesis ratio was 89.1% and the reaction product contained 81.1% of propylene glycol monostearate and 8.0% of propylene glycol distearate.

Example 34

0.81 g (3.54 mM) of myristic acid, 0.81 g (10.6 mM) of propylene glycol, 2.5 ml of tertiary butyl alcohol, 2.5 ml of diacetone alcohol, 100 mg of immobilized lipase sp-382 and 1 g of molecular sieve 3A were charged into 30 ml volume Erlenmeyer flask and then reacted at 50- for 6 hours under shaking. As a result, the ester synthesis ratio was 92.8% and the reaction product contained 90.7% of propylene glycol monomyristate and 2.1% of propylene glycol dimyristate.

Example 35

Heat resistance test for various lipases

After dissolving 50 mg of each powdery lipase shown in Table 3 into 0.4 ml of a phosphoric acid buffer (0. 1M, pH 7), it was heated at 70°C for 30 min. After cooling the solution with cold water, it was diluted with the same buffer solution to prepare an enzyme solution sample.

Then, 1 ml of the enzyme solution sample was mixed with 6 ml of a substrate solution (40% olive oil emulsion solution) and an enzymatic reaction was conducted at 30°C for 30 min. After the reaction was over, 10 ml of ethanol/acetone/0. 02 mol EDTA (volume ratio = 1/1/1) solution was added to terminate the enzymatic reaction. As the blank, not-heated aqueous lipase solution was used and the enzymatic reaction was conducted in parallel.

After the enzymatic reaction was over, it was titrated with 0.025N sodium hydroxide solution, the enzymatic activity was calculated based on the amount of free fatty acid and the relative activity before and after the heating was determined by using the following equation:

$$\text{Relative activity (\%)} = \frac{\text{Enzymatic activity after heating}}{\text{Enzymatic activity before heating}} \times 100$$

Ester exchanging performance of various immobilized lipases in the solution

To a mixture of 1.56 g (1.79 mM) of triolein and 0.99 g (10.74 mM) of glycerol, were added 10 ml of tertiary butyl alcohol and, further, 100 mg of each kind of lipases shown in Table 1 immobilized to a porous acrylic resin and they were reacted at 60°C for 4 hours under stirring. After the reaction was over, it was centrifugaly separated to remove the immobilized lipase.

Then, 20 $\mu$l of the reaction filtrate was charged into 1 ml of a screw tube, to which were added 30 $\mu$l of pyridine and, further, 50 ml of pyridine incorporated with n-tetradecane as an internal standard material (20 mg/ml) and 200 $\mu$l of bis(trimethylsilyl)trifluoroacetoamide as a silylating agent and they were reacted being kept stationary at 70 to 80°C for 30 min. 1 $\mu$l of the reaction solution was analyzed on gas chromatography and the percent by weight of glycerol monoester, diester and triester in the oil were measured to calculate the glycerol monoester content as the ester exchange ratio.

$$\text{Ester exchange ratio (\%)} = \frac{\text{Amount of monoester (mg)} \times 100}{\text{Amount of monoester (mg)} + \text{Amount of diester (mg)} + \text{Amount of triester (mg)}}$$

The foregoing results are shown together in Table 3.

Table 3

| Lipase | | Retative activity (%) | Ester exchange rate (%) |
|---|---|---|---|
| Origin | Commercial name (manufacturer's name) | | |
| Candida antarctica | sp-382 (NOVO Co.) | 100 | 89 |
| Mucor miehei | Lipozyme (NOVO Co.) | 97 | 75 |
| Mucor sp | sp-225 (NOVO Co.) | 4 | 7 |
| Mucor sp | sp-356 (NOVO Co.) | 7 | 5 |
| Mucor sp | sp-400 (NOVO Co.) | 0 | 4 |
| Rhizodpus delemer | Lipase (Seikagaku Kogyo Co., Ltd.) | 13 | 11 |
| - | Lipase (Nagase Industrial Co., Ltd.) | 12 | 0 |
| - | Lipase (Toyo Jozo Co., Ltd.) | 5 | 8 |
| - | Talipase (Tanabe Seiyaku Co., Ltd.) | 0 | 0 |
| Candida rugosa | Lipase MY (Meito Sangyo Co., Ltd.) | 18 | 0 |
| Candida rugosa | Lipase DF (Meito Sangyo Co., Ltd.) | 23 | 0 |
| Alcaligenes sp | Lipase PL (Meito Sangyo Co., Ltd.) | 16 | 12 |
| Pseudomonas fluorescens | Lipase P (Amano Seiyaku Co., Ltd.) | 31 | 6 |
| Penicillium aurantiogriseum | Lipase G (Amano Seiyaku Co., Ltd.) | 5 | 0 |
| Aspergillus niger | Lipase AP (Amano Seiyaku Co., Ltd.) | 10 | 0 |
| Rhizopus sp | Saiken (Osaka Saikin Laboratory) | 0 | 0 |
| Rhizopus japonicus | Saiken (Osaka Saikin Laboratory) | 0 | 0 |
| Arthrobacter sp | BSL (Godo Shusei Co., Ltd.) | 12 | 0 |
| Rhizopus arrhizus | Lipase (Sigma Co.) | 8 | 0 |
| Porcine pancreas | Lipase (Sigma Co.) | 0 | 0 |

From the result shown in Table 3, it can be seen that Lipase sp-382 derived from Candida antarctica and Lipozyme derived from Mucor miehei, being immobilized, have excellent heat resistance and can attain a high ester synthesis ratio at 60° C, which is a relatively high reaction temperature for the lipase reaction and in a shorter period of time.

Example 36

1. 60 g (1. 79 mM) of tristearin, 10 ml of diacetone alcohol and 100 mg of immobilized lipase sp382 were charged into 30 ml volume Erlenmeyer flask, to which glycerol was added each in an amount of 0.33 g (3.58 mM), 0. 99 g (10.74 mM), 1. 65 g (17. 90 mM), respectively and they were reacted at 50° C for 5 hours. After the reaction was over, monostearin and distearin formed in the ester exchanging reaction and unreacted tristearin were analyzed on gas chromatography and each of the ingredient contents was determined. The results are shown in Table 4.

Table 4

| Glycerol/tristearin (molar ratio) | Ingredient ratio (%) | | |
|---|---|---|---|
| | Monostearin | Distearin | Tristearin (unreacted product) |
| 2 | 73.3 | 25.3 | 1.4 |
| 6 | 90.1 | 9.8 | 0 |
| 10 | 90.4 | 9.6 | 0 |

From the results of Table 4, it can be seen that the content of monostearin is increased along with the increase in the molar ratio of glycerol/tristearin.

Fatty acid (stearic acid) formed by hydrolysis was less than 0.5% in each of the cases.

Example 37

0.99 g (10.74 mM) of glycerol, 7 ml of tertiary butyl alcohol and 100 mg of immobilized lipase sp-382 were charged into 30 ml volume Erlenmeyer flask, to which glyceride oils and fats shown in Table 5 were added each by 1.5 g respectively and they were reacted at 70° C for 3 hours. After the reaction was over, glycerol monoester and glycerol diester formed in the ester exchanging reaction and unreacted glycerol triester (glyceride oil and fat) were analyzed on gas chromatography to determine each of the ingredient contents. The results are shown in Table 5.

From the results of Table 5, it can be seen that the monoester can be produced in an extremely shorter period of time for the enzymatic reaction and at a high content in each of the cases of using animal and vegetable oils and fats by using the process of the present invention.

Table 5

| Oil and fat | Ingredient ratio (%) | | |
|---|---|---|---|
| | Glycerol monoester | Glycerol diester | Glycerol triester (unreaction product) |
| Safflower oil | 87.4 | 12.6 | 0 |
| Soybean oil | 83.7 | 12.9 | 3.4 |
| Soybean germ oil | 84.1 | 13.0 | 2.9 |
| Wheat oil | 85.8 | 12.5 | 1.7 |
| Sesame oil | 87.2 | 11.9 | 0.9 |
| Corn oil | 86.3 | 12.7 | 1.3 |
| Rape seed oil | 85.5 | 10.8 | 3.7 |
| Olive oil | 84.3 | 12.5 | 3.2 |
| Castor oil | 85.8 | 11.8 | 2.4 |
| Palm oil | 89.0 | 11.0 | 0 |
| Palm hernel oil | 90.2 | 9.8 | 0 |
| Coconut oil | 88.9 | 11.1 | 0 |
| Cotton seed oil | 88.6 | 11.4 | 0 |
| Beef tallow | 89.7 | 10.3 | 0 |
| Lard | 89.1 | 10.7 | 0 |
| Horse tallow | 87.5 | 10.9 | 1.6 |
| Mackerel oil | 82.3 | 13.4 | 4.3 |
| Sardine oil | 85.1 | 12.2 | 2.7 |
| Saury oil | 83.9 | 12.6 | 3.5 |

Example 38

1.56 g (1.78 mM) of triolein, 0.81 g (10.74 mM) of propylene glycol, 10 ml of 2,4-dimethyl-3-pentanol and 100 mg of immobilized lipase (Lipozyme) were charged into 30 ml volume of Erlenmeyer flask and they were reacted 50°C for 6 hours. After the reaction was over, monoester and diester formed in the ester exchanging reaction and unreacted triester (triolein) were analyzed.

As a result, glycerol monoolein was 25.0% propylene glycol monoolein was 66.0%, glycerol diolein was 9.1% propylene glycol diolein was 0% and triolein was 0%. Oleic acid was not detected.

Example 39

1.56 g (1.79 mM) of triolein, 25 ml of tertiary butyl alcohol and 100 mg of immobilized lipase sp-382 were charged into 30 ml volume Erlenmeyer flask, to which 1.77 g (10.74 mM) of diglycerol, 3.37 g (10.74 mM) of tetraglycerol and 8.09 g (10.74 mM) of decaglycerol were added respectively and they were reacted at 60°C for 6 hours. After the reaction was over, monoester and diester formed in the ester exchanging reaction and unreacted triester (triolein) were analyzed. The results are shown in Table 6.

Table 6

| Polyglycerol (average polymerization degree) | Ingredient ratio (%) | | | | |
|---|---|---|---|---|---|
| | Glycerol monooleate | Polyglycerol monooleate | Glycerol dioleate | Polyglycerol dioleate | Triolein (unreacted products) |
| Diglycerol (2) | 14.2 | 80.0 | 2.0 | 5.9 | 0 |
| Tetraglycerol (4) | 11.2 | 78.7 | 1.9 | 6.3 | 1.9 |
| Decaglycerol (10) | 14.8 | 70.2 | 1.5 | 4.8 | 8.7 |

Example 40

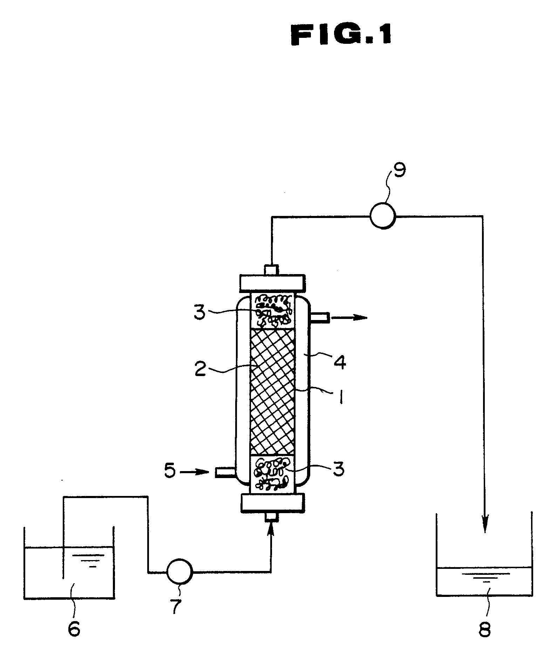

As shown in Fig. 1, 1 g of immobilized lipase sp-382 (denoted by 2 in the figure) was packed in a jacketted column 1 with 6 mm inner diameter and 110 mm length to prepare an enzyme column. Glass wools 3,3 were packed respectively in the inlet and the outlet of the enzyme column, which was fixed vertically and warm water 5 at 50° C was circulated through a jacket portion 4 to keep the temperature of the enzyme column at 50° C.

Then, a substrate solution 6 at a glycerol concentration of 100 g/l and an olive oil concentration of 150 g/l was introduced by using tertiary butyl alcohol as a solvent into the enzyme column from below the enzyme column by using a pump 7 at a flow rate of 50 ml/hr, passed through the enzyme column with a stay time of 4 minutes to conduct an enzymatic reaction. The resultant reaction solution 8 was drained by a pump 9 into a storage vessel.

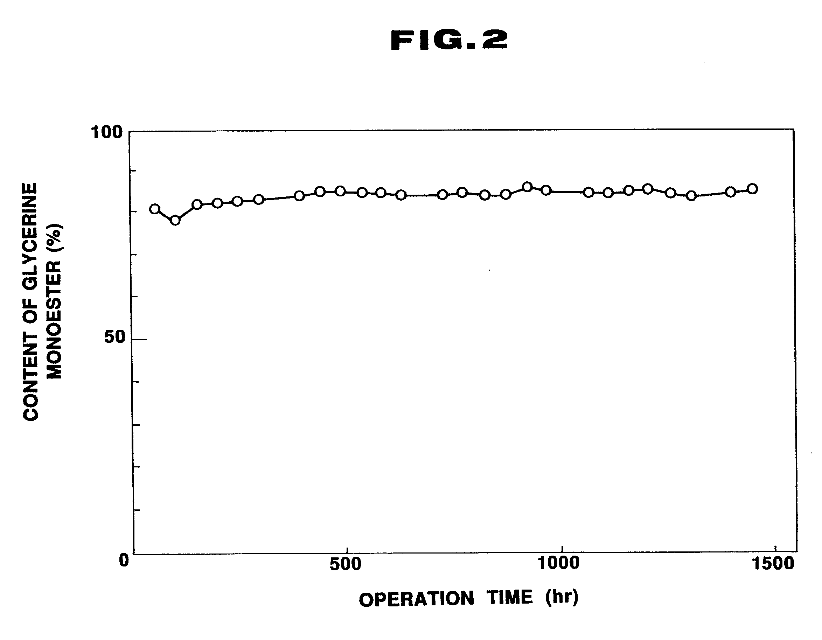

Meanwhile, the reaction solution 8 was periodically sampled and monoester content was measured by gas chromatography. The results are shown in Fig. 2.

From the results of Fig. 2, it was observed that the amount of glycerine monoester in the oil (reaction products) was kept at a content of about 85% even after elapse of about 1,500 hours. This proves that the enzyme activity was kept stable in the enzyme column even after a long period of time.

**Claims**

1. A process for producing a polyol fatty acid monoester in which a thermostable immobilized lipase is acted under the presence of a secondary alcohol and/or tertary alcohol on a mixture of a saturated or unsaturated fatty acid having 6 to 22 carbon atoms or an ester of said fatty acid with a lower alcohol having 1 to 3 carbon atoms and a polyol selected from glycerol and its derivatives, polyglycerol and its derivatives, ethylene glycol, polyethylene glycol, propylene glycol, and polypropylene glycol.

2. The process of claim 1 wherein the thermostable immobilized lipase has such heat resistance as possessing greater than 40% of the residual activity after dissolving 50 mg of a lipase powder into 0.4 ml of a phosphoric acid buffer (0.1M, pH4) and then heating at 70° C for 30 min.

3. The process of claim 1 or 2 wherein the alcohol selected from the group consisting of the secondary alcohol and tertiary alcohol is selected from 2,4-dimethyl-3-pentanol, 2,6-dimethyl-4-heptanol, tertiary butyl alcohol, tertiary amyl alcohol, diacetone alcohol, 3-methyl-3-pentanol, 3-ethyl-3-pentanol, 3-propyl-3-pentanol, 2-methyl-2-hexanol and 2-ethyl-2- hexanol.

4. The process of any of claims 1 to 3 wherein the amount of the secondary or tertiary alcohol is from 10 to 99% by weight of the reaction system.

5. The process of any of claims 1 to 4 wherein the mixing ratio of the fatty acid or its ester and the polyol is from 0. 1 to 10 mol based on one mol of the fatty acid or its ester.

6. The process of any of claims 1 to 5 wherein the reaction is conducted at a temperature of higher than 40° C.

7. A process for producing a polyol fatty acid monoester in which an ester exchanging reaction is conducted by acting a thermostable immobilized lipase under the presence of a secondary alcohol and/or tertiary alcohol on a mixture of glyceride oil or fat having a saturated or unsaturated fatty acid having 6 to 22 carbon atoms as a constituent fatty acid and a polyol selected from glycerol and its derivatives, polyglycerol and its derivatives, ethylene glycol, polyethylene glycol, propylene glycol, and polypropylene glycol.

8. The process of claim 7 wherein the thermostable immobilized lipase has such heat resistance as possessing greater than 40% of the residual activity after dissolving 50 mg of a lipase powder into 0.4 ml of a phosphoric acid buffer (0.1M, pH4) and then heating at 70°C for 30 min.

9. The process of claim 7 or 8 wherein the alcohol selected from the group consisting of the secondary alcohol and the tertiary alcohol is selected from 2,4-dimethyl-3-pentanol, 2,6-dimethyl-4-heptanol, tertiary butyl alcohol, tertiary amyl alcohol, diacetone alcohol, 3-methyl-3-pentanol, 3-ethyl-3-pentanol, 3-propyl-3-pentanol, 2-methyl-2-hexanol and 2-ethyl-2-hexanol.

10. The process of any of claims 7 to 9 wherein the amount of the secondary or tertiary alcohol is from 10 to 99% by weight of the reaction system.

11. The process of any of claims 7 to 10 wherein the mixing ratio of the glyceride fat or oil and the polyol from 0.2 to 20 mol based on one mol of the fatty acid or its ester.

12. The process of any of claims 7 to 11 wherein the reaction is conducted at a temperature of higher than 40°C.

# FIG.1

# FIG.2

CONTENT OF GLYCERINE MONOESTER (%)

OPERATION TIME (hr)